# EUROPEAN PATENT APPLICATION

(11) **EP 1 736 154 A1**
(43) Date of publication of application: **27.12.2006**
(21) Application number: 05013659.7
(22) Date of filing: 24.06.2005
(51) Int. Cl.: A61K 31/35, A61P 25/28, A61P 35/00

(54) **Use of PP-1 inhibitors to prevent missplicing events**

(71) Applicant: Friedrich-Alexander-Universität Erlangen-Nürnberg, 91054 Erlangen (DE)
(72) Inventor: Stamm, Stefan, 91052 Erlangen (DE); Novoyatleva, Tatyana, 91052 Erlangen (DE)
(74) Representative: Becker, Philippe

(57) **Abstract**

The present invention relates to methods and compositions for regulating splicings in mammalian cells. More particularly, the invention relates to the use of PP1 inhibitors to prevent abnormal splicings in various pathological situations. The invention may be used to prevent or treat various diseases, including degenerative diseases and cancers.

## Description

The present invention relates to methods and compositions for regulating splicing in mammalian cells. More particularly, the invention relates to the use of PP1 inhibitors to prevent abnormal splicings in various pathological situations. The invention may be used to prevent or treat various diseases, including degenerative diseases and cancers.

### Background

Sequencing of various eukaryotic genomes demonstrated that a surprisingly small number of genes generate a complex proteome. For example, the estimated 20,000-25,000 human protein-coding genes give rise to 100-150,000 mRNA variants as estimated by EST comparison. Array analysis shows that 74% of all human genes are alternatively spliced (Johnson et al., 2003) and a detailed array-based analysis of chromosome 22 and 21 suggests that every protein coding gene could undergo alternative splicing (Kampa et al., 2004). Extreme examples illustrate the potential of alternative splicing: the human neurexin 3 gene could form 1728 transcripts (Missler and Sudhof, 1998) and the Drosophila DSCAM gene could give rise to 38016 isoforms, which is larger that the number of genes in Drosophila (Celotto and Graveley, 2001).

Proper splice site selection is achieved by binding of protein and protein:RNA complexes (trans-factors) to weakly defined sequence elements (cis-factors) on the pre-mRNA. Binding of the trans-factors occurs cotranscriptionally and prevents the pre-mRNA to form RNA : DNA hybrids with the genomic DNA. RNP complexes forming around exons promote binding of U2AF and U 1 snRNP at the 3' and 5' splice sites respectively, which marks the sequences to be included in the mRNA. Sequences located in exons or the flanking introns can act as splicing silencers or enhancers. All cis-elements can only be described as consensus sequences that are loosely followed (Black, 2003) and in general, they bind only weakly to trans-acting factors. The action of the cis-elements depend on other surrounding elements and due to this sequence context, the same sequence can either promote or inhibit exon inclusion (Carstens et al., 1998). In order to achieve the high fidelity of splice site selection, multiple weak interactions are combined (Maniatis and Reed, 2002; Maniatis and Tasic, 2002) and as a result of this combinatorial control, splice site selection is influenced by multiple factors (Smith and Valcarcel, 2000). This combinatorial control is mirrored in the complex composition of splicing regulatory complexes that often combine overlapping enhancing and silencing parts that collaborate to regulate exon usage (Singh et al., 2004b) (Pagani et al., 2003).
The usage of alternative exons changes during development or cell differentiation both *in vivo* and in cell cultures. Furthermore, numerous external stimuli have been identified that change alternative splicing patterns. In most cases, these changes are reversible, indicating that they are part of a normal physiological response (Stamm, 2002).

Since alternative splicing plays such an important role in gene expression, it is not surprisingly that an increasing number of diseases are caused by abnormal splicing patterns. There is a positive correlation between the number of splice sites and the likelihood of a gene to cause a disease, suggesting that many mutations causing diseases may actually disrupt the splicing pattern of a gene (Lopez-Bigas et al., 2005). The disease causing mechanism can be subdivided into changes in cis and trans-factors. Changes in cis-factors are caused by mutation in splice sites, silencer and enhancer sequences and through generation of novel binding sites in triplet repeat extensions. Alterations in trans-acting factors are frequently observed in tumor development, where the concentration and ratio of individual trans-acting factors changes. A misregulation of pre-mRNA processing causes human diseases, for example Cystic Fibrosis, Spinal muscular atrophy and Alzheimer's disease. The inclusion of exons is regulated by SR and SR-like proteins that bind to the exonic RNA and form exon recognition complexes. The SR-like protein tra2-betal is an important component of numerous exon recognition complexes. The interaction between tra2-betal and other proteins is regulated by phosporylation.

So far, treatment of diseases caused by missplicing has been attempted by three strategies: transfer of genes (Garcia-Blanco et al., 2004.) or nucleic acids that act directly on genes and low molecular weight drugs.

### Gene transfer methods

Antisense nucleic acids can suppress point mutations and promote the formation of the normal gene products. Special chemistries were devised to prevent RNAseH mediated cleavage of the RNA and to lower toxicity (Sazani and Kole, 2003). Antisense oligonucleotides have been used to modify U7 snRNA which results in the nuclear accumulation of the oligonucleotides in stable U7nRNP complexes (Asparuhova et al., 2005). Oligonucleotides have been used to target cryptic splice sites that are activated in beta-thalassemias (Lacerra et al., 2000), to suppress exon usage in Duchenne muscular dystrophy (Mann et al., 2001) and block HIV replication (Liu et al., 2004).
The antisense approach was further developed in ESSENCE (exon-specific splicing enhancement by small chimeric effectors). ESSENCE uses bifunctional reagents that contain a peptide effector domain and an antisense targeting domain. The effector domains of these protein-nucleic acids were arginine-serine (RS) repeats that mimic the effect of SR-proteins (Cartegni and Krainer, 2003).
Related to ESSENCE is the use of bifunctional oligonucleotides in TOES (targeted oligonucleotide enhancer of splicing), where a part of the oligonucleotide binds to an SR-protein, which promotes exon inclusion (Skordis et al., 2003). Several RNA based approaches have been tested in cell culture. They include use of RNAi to suppress unwanted isoforms (Celotto and Graveley, 2002), spliceosome-mediated RNA trans-splicing (SmaRT) to correct factor VIII deficiency in a mouse model (Chao et al., 2003) and ribozymes that use trans-splicing to replace defective p53, beta-globin mRNA and a chloride channel in cell culture (Lan et al., 1998; Watanabe and Sullenger, 2000; Rogers et al., 2002).

### Low molecular weight drugs

It is well known that small molecules can interact with RNA and this principle is used by several RNA binding antibiotics, such as gentamicin, chloramphenicoland tetracycline (Xavier et al., 2000). Therefore, several chemical screens were performed to identify small molecular weight molecules that interfere with splice site selection. It was found that (-)-epigallocatechin gallate (EGCG), a polyphenol and component of green tea (Anderson et al., 2003), as well as kinetin and the related bunzyladcnine, a plant hormone (Slaugenhaupt et al., 2004), promote correct splice site usage in the IKAP gene, involved in Familial dysautonomia. An important class of these components are histone deacetylase inhibitors, such as sodium butyrate and valproic acid that have been used to increase the correct level of SMN2 splicing (Chang et al., 2001; Brichta et al., 2003). SMN2 splicing was also influenced by the phosphatase inhibitor sodium vanadate (Zhang et al., 2001) and the cytotoxic anthracycline antibiotic aclarubicin (Andreassi et al., 2001) and the nonsteroidal anti-inflammatory drug indoprofen (Lunn et al., 2004). Indole derivatives were also found to act on specific SR-proteins that regulate specific ESE sequences (Soret et al., 2005). Since these substances block HIV replication by blocking early viral splicing events, they open the intriguing possibility of a specific pharmacological treatment of splicing disorders. However, a major disadvantage of most of the inhibitors is their low specificity.

### Summary of the Invention

The present invention now provides a novel approach for treating diseases caused by improper or aberrant splicings in mammalian cells and organisms.

In particular, the present invention shows, for the first time, that Protein Phosphatase 1 ("PP1") regulates the activity of splicing enhancer complexes that contain the proteins tra2-beta1, SF2/ASF, SRp30c and p54. The invention more specifically shows that PP1 changes the phosphorylation status of these proteins, which alters the formation of exon recognition complexes and influences splice site selection. Accordingly, the present invention shows that PP1 represents a valuable target for controlling splicing in a number of pathological conditions, particularly in diseases which involve an improper pre-mRNA processing regulated by a tra2-beta1-containing splicing enhancer complex. Examples of such improper splicing events that are regulated by tra2-beta1-containing splicing enhancer complex include alternative splicing of SMN2, which is involved in spinal muscular atrophy, alternative splicing of CFTR 84nt exon, which is involved in Cystic fibrosis, and alternative splicing of tau exon 10, which is involved in Alzheimer's disease and tauopathies. The present invention also demonstrates that PP1 activity can be blocked by drugs like tautomycin, tautomyceitin, fostriecin, Calyculin A, Microcystin-LR, Cantharidin and Thyrsiferyl-23-acetate. Treatment of cells with these drugs alters splice site selection, which removes the cause of spinal muscular atrophy, tauopathies and forms of cystic fibrosis.

Accordingly, an object of this invention relates to the use of a PP1 inhibitor for the manufacture of a medicament (drug) to treat a disease caused by improper pre-mRNA processing.

A further object of this invention resides in a method of treating a disease caused by improper pre-mRNA processing, comprising administering to a subject in need thereof an amount of a PP1 inhibitor effective at regulating improper pre-mRNA processing, thus treating said disease.

As will be discussed below, the improper pre-mRNA processing is preferably regulated by a tra2-beta1-containing splicing enhancer complex. The invention may be used to treat a variety of diseases, including neuro-degenerative diseases, tauopathies, cancers and cystic fibrosis.

An other object of this invention relates to the use of a PP1 inhibitor to influence splice site selection in a mammalian cell in vitro or ex vivo, as well as to a corresponding method.

A further particular object of this invention is directed to the use of tautomycin for the manufacture of a medicament (drug) to treat spinal muscular atrophy, tauopathies or Cystic fibrosis, as well as to a corresponding method of treatment.

The invention also relates to the use of a PP1 inhibitor for the manufacture of a medicament (drug) to treat the effects of cancer that are caused by the selection of wrong splice sites, as well as to a corresponding method of treatment.

A further aspect of this invention is a method of preventing or inhibiting (the effect(s) of) an improper pre-mRNA splicing in a pathological condition, comprising administering to a subject in need thereof an amount of a PP1 inhibitor effective at preventing or inhibiting said pre-mRNA splicing.

As will be discussed in detail below, the PP1 inhibitor is preferably a selective PP1 inhibitor. It is most preferably a small drug inhibitor, selected for instance from Tautomycin, Tautomycetin, Fostriecin. Microcystin-LR, Calyculin A, Cantharidin, Thyrsiferyl-23-acetate, Nodularin. Dephostatin, 3,4-Dephostatin, Fumonisin B1 and Motuporin, salts and derivatives thereof, as well as any combination(s) thereof. Also, the PP1 inhibitor may be used in combination with at least one additional active agent or treatment.

The invention also relates to methods of selecting or identifying or improving drugs that regulate pre-mRNA processing, the method comprising a step of determining whether a drug candidate inhibits PP 1.

A further embodiment of this invention relates to a method of selecting or identifying or improving a drug candidate for treating cancer or a CNS disorder or a tauopathy or Cystic Fibrosis, the method comprising a step of determining whether said drug candidate inhibits PP 1.

Preferably, the method is performed in vitro or ex vivo, in binding or cell-based assays. The method typically comprises contacting the drug candidate with PP1 or a portion thereof, and determining whether the drug candidate binds to PP1 or said portion thereof. Most preferably, the method is performed in the presence of tra2-betal or a PP1-binding domain thereof, and comprises determining whether the drug candidate interferes with the binding of PP1 to tra2-beta1 or said binding domain thereof

The invention may be used in various mammalian subjects, including human subjects, at various stages of a disease, either for curative or preventive treatment.

### Legend to the Figures

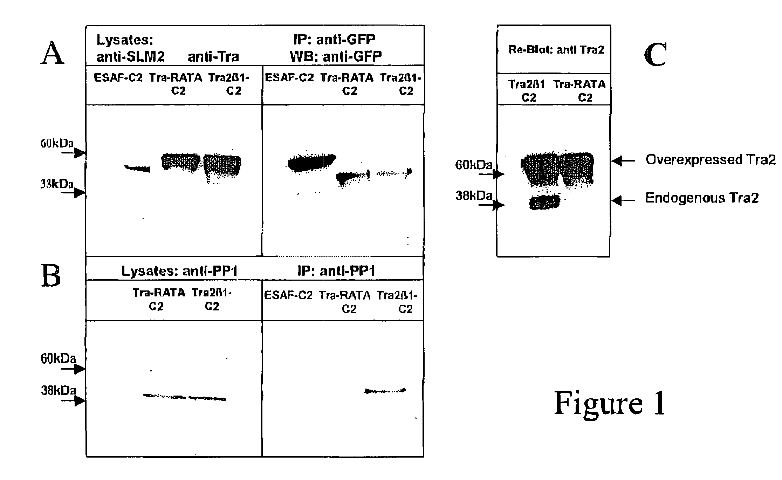

Figure 1: Tra2-betal interacts with PP1c
   This figure shows the interaction of Tra2β1-C2 and Tra2B1-(RVDF-RATA)-C2 with HA-PP1. EGFP-Tra2-betal was cotransfected with HA-PP1. EGFP and HA are enhanced fluorescent protein and haemagglutinin tags, respectively. Tra2-beta1 RATA is a mutant that does not bind to PP1 (RVDF -> RATA). (A) Immunoprecipitation was performed with anti -GFP and the successful immunoprecipitation verified by Western blot using anti GFP (B). The reblot was performed with anti-PP1 antisera, detecting only PP 1 signal with the immunoprecipitation of anti tra2-betal. The unrelated protein rSLM-2 serves as a negative control.
   (C): Reblotting the immunoprecipitates with anti tra2-betal reveals that wild type tra2-betal, but not the tra2-betal RATA mutant binds to endogenous tra2-betal.

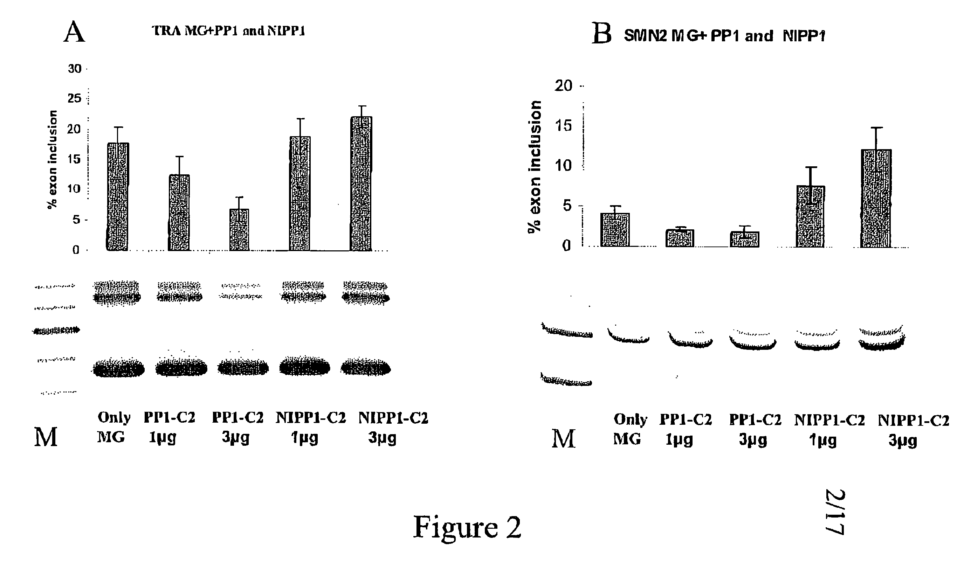

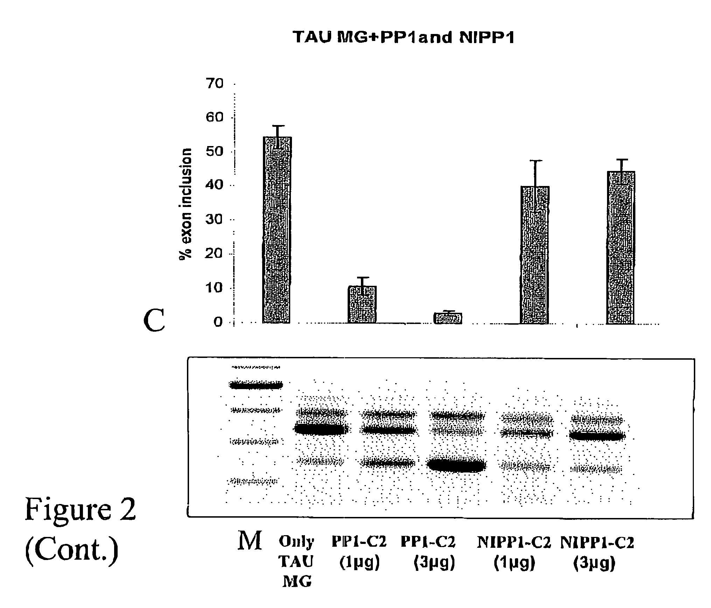

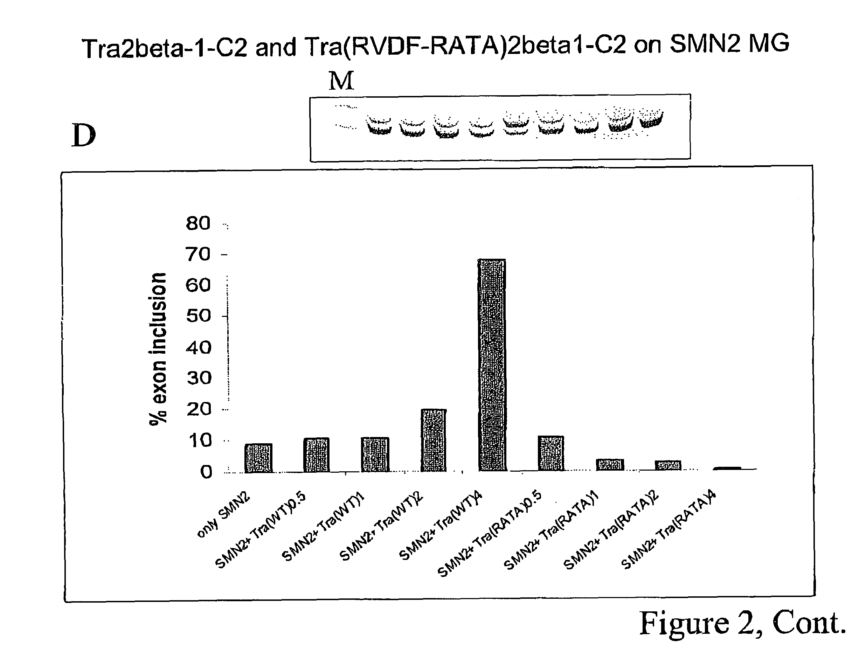

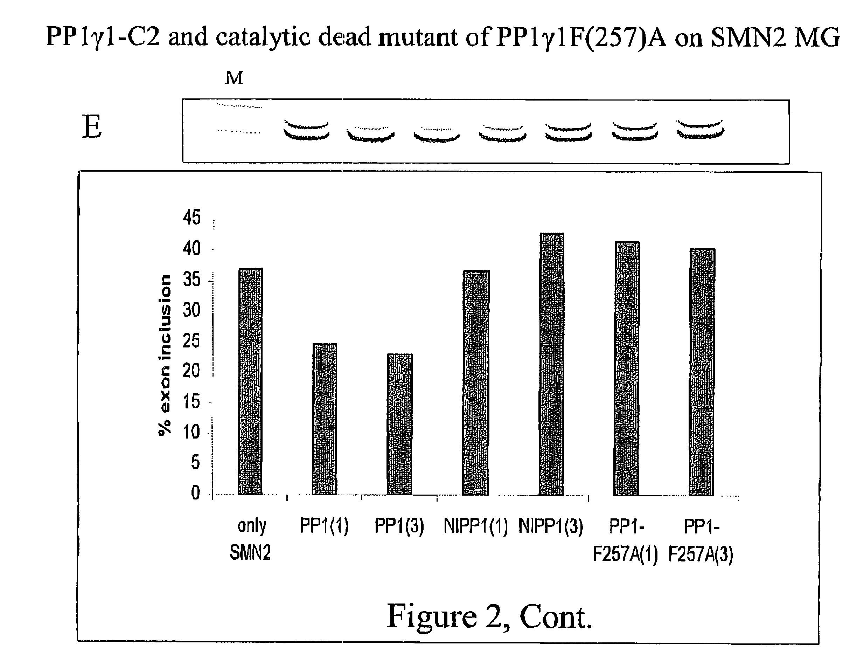

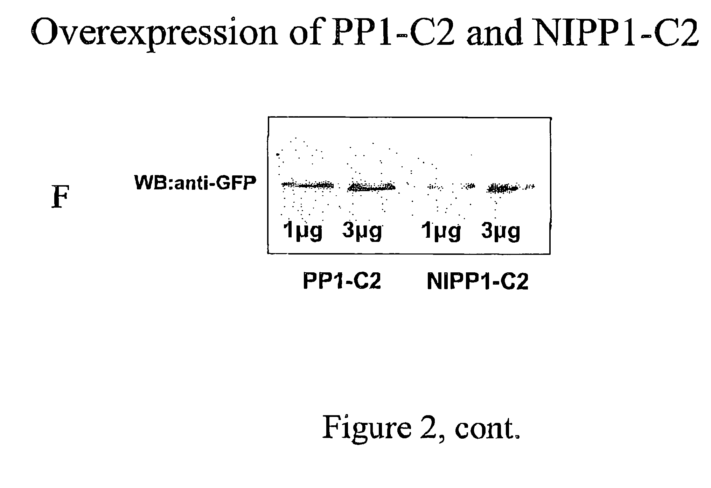

Figure 2: PP1 regulates the activity of tra2-beta1 dependent splicing enhancer.
   The appropriate splicing reporter minigene was cotransfected with either an increasing amount of EGFP-PP1 (PP1-C2) or EGFP-NIPP (EGFP-C2). After cotransfection, RNA was isolated and analysed by reverse transcription. A representative ethidiume bromide stained gel is shown below the statistical analysis of at least three independent experiments.
   A: The inclusion of tra2-beta1 exon II was tested
   B: The SMN2 minigene, exon 7 was tested
   C: The Tau minigene, exon 10 was tested In panels D and E, the tra2-beta1mutant unable to bind to PP1 and a catalytically dead mutant of PP1 was used in in vivo minigene assays with the SMN2 minigene. Both mutants have no significant effect on exon 7 inclusion.
   F: The increasing amounts of the proteins PP1-C2 and NIPP1-C2 were verified by Western Blot
Figure 3: The PP1 inhibitor tautomycin promotes inclusion of tra2-beta1 dependent enhancers, Cells were transfected with the tau or SMN reporter minigenes and treated with 300 or 600 nM tautomycin. Splicing patterns were analysed by RT-PCR.
   A: Analysis of the tau exon 10 minigene
   B: Analysis of the SMN2-minigene
   C: Analysis of the SMN2-minigene with different concentrations of tautomycin
Figure 4: Tautomycin promotes SMN Protein formation in human fibroblasts
   Human fibroblasts from SMA patients were treated with 3 mM valproic acid or 40 nM Tautomycin for 2 to three days. An antibody against SMN was used to detect the formation of SMN that is absent in patient fibroblasts. GAPDH was used as a loading control. Fibroblasts from healty individuals were used as a control. Numbers in parenthesis indicate dilution of the antisera.

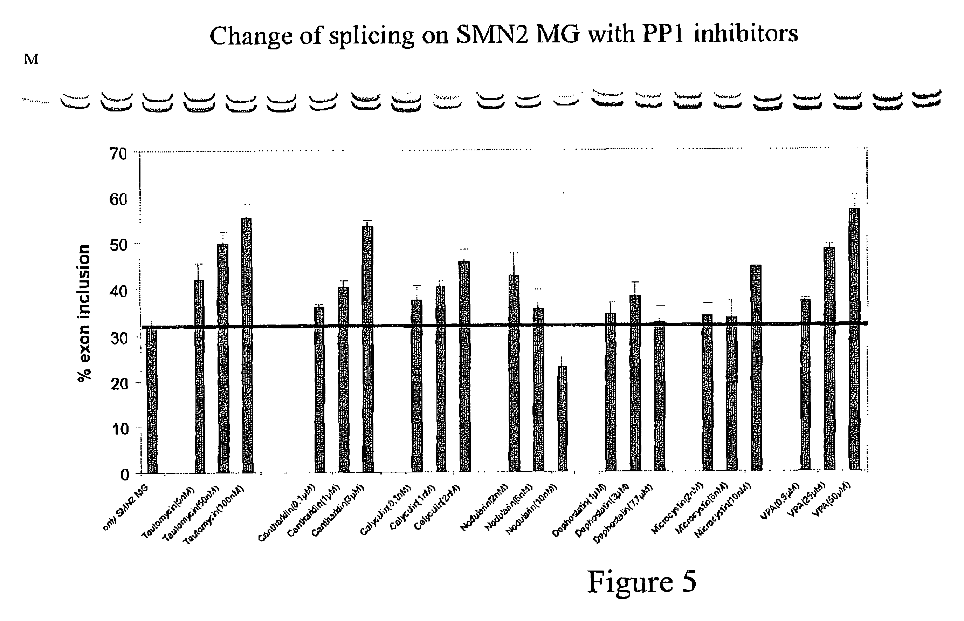

Figure 5: Comparison of different PP1 inhibitors
   HEK293 cells were transfected with the SMN2 minigene and treated with the inhibitors and their concentration listed below. Treatment was overnight. Error bars represent standard deviation from at least three independent experiments.

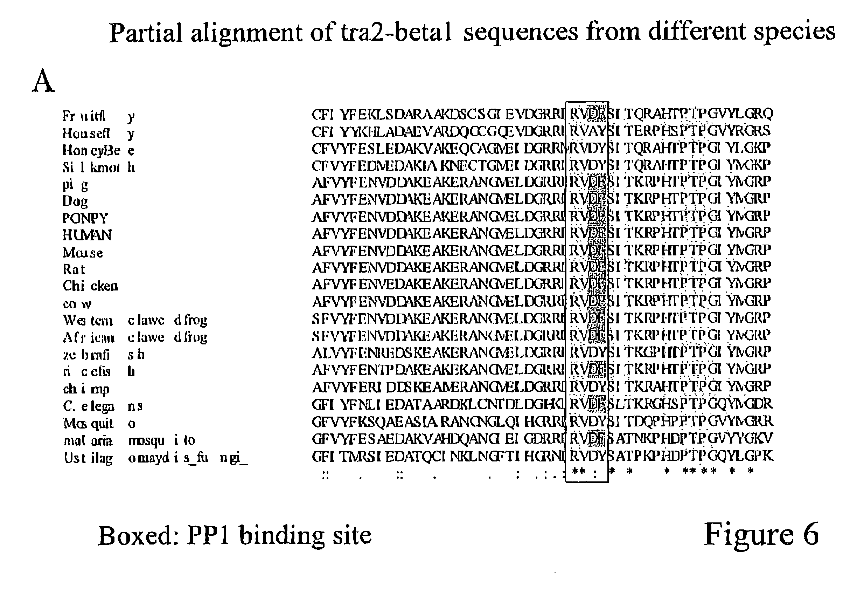

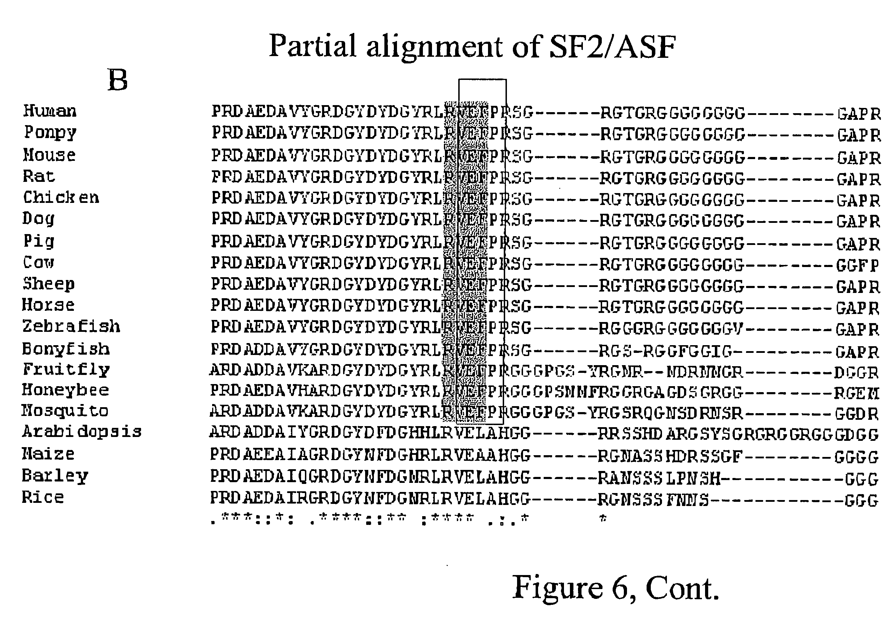

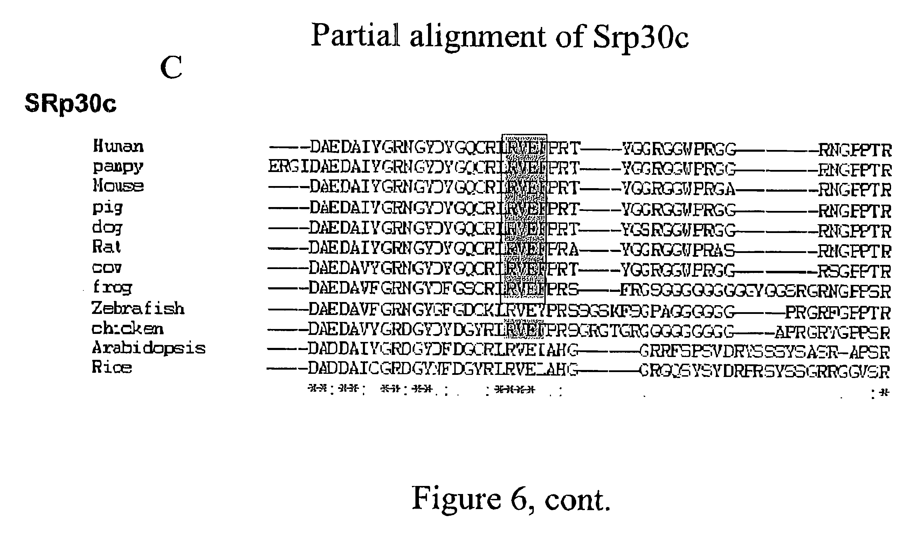

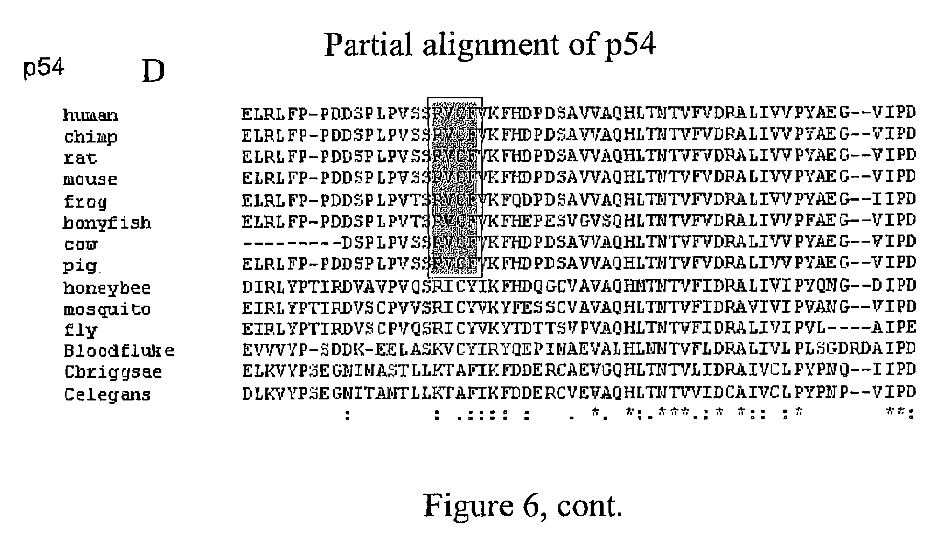

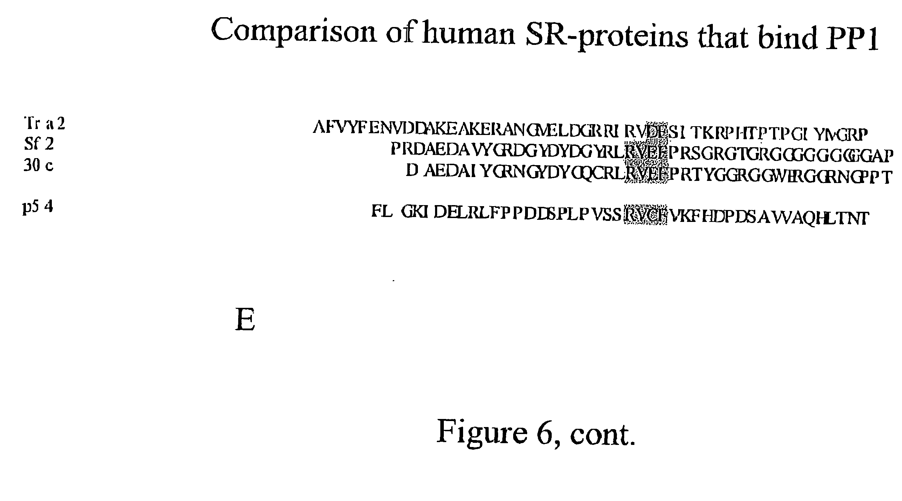

Figure 6: Conservation of the RVXF binding motif in splicing factors
   A. Partial alignment of tra2-betal sequences from different species. Residues that are fully conserved between the species are shown in yellow
   B. Partial alignment of SF2/ASF between different species
   C. Partial alignment of SRp30c between different species
   D. Partial alignment of SRp54 between different species
   E. Comparison of human SR-proteins that bind PP 1, only the sequence around the PP 1 binding site are shown.

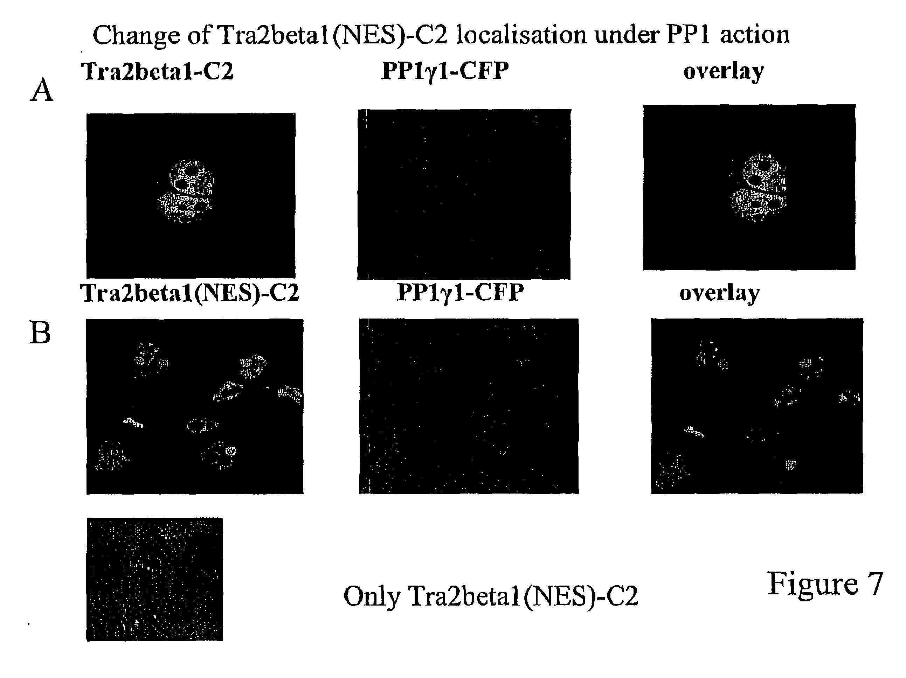

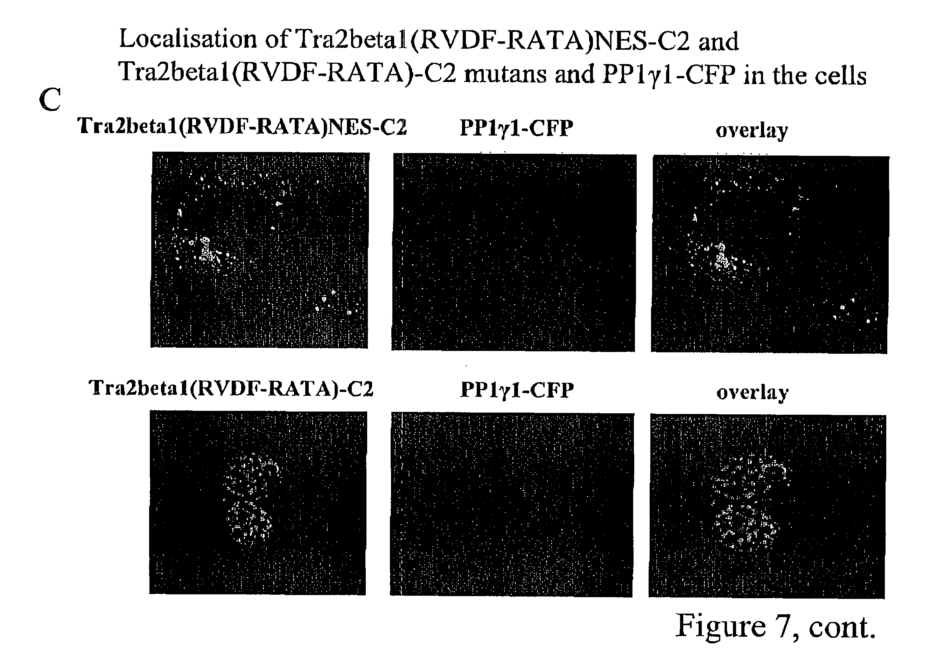

Figure 7: A Tra2beta 1-C2 and PP1-CFP in cells
   B Change of Tra(NES)-C2 under the action of PP1
   C Localisation of Tra2beta1 (RVDF-RATA)-C2 and Tra2beta1 (RVDF-RATA)-C2 with PP1-CFP in the cell.

### Detailed description of the Invention

The present invention stems from the unexpected and important discovery that PP1 regulates splice site selection in various pathological conditions, and thus represents a highly valuable target for therapeutic intervention as well as for drug development.

### Protein Phosphatase 1 (PP1)

Within the context of the present invention, the term protein phosphatase 1 designates a mammalian polypeptide that regulates protein de-phosphorylation. PP1 has been isolated and cloned from various species, including human, and the sequence of a wild-type PP1 is available in protein libraries under accession number given in Table1 and Table 2. PP1 more specifically refers to the catalytic subunits of protein phosphatase 1. There are three subunits derived from related genes with biochemically undistinguishable identities. Their HUGO names are PPP1CA, PPP1CB, PPP1CC for protein phosphatase catalytical subunit alpha, beta, and gamma. The identifiers for cDNA are given in the RefSeq Genbank number and for protein in the Uniprot Accession numbers.

**Table 1: Acession numbers of human PP1 isoforms**

| HUGO | RefSeq | UniProt | reference |
|---|---|---|---|
| PPP1CA | NM_002708 | P08129 | (Barker et al., 1994) |
| PPP1CB | NM_002709 | P37140 | (Barker et al., 1994) |
| PPP1CC | NM_002710 | P36873 | (Song et al., 1993) |

The term PP1 also includes any naturally-occurring variants of the sequence as depicted above, such as variants resulting from polymorphisms, splicings, mutations, etc. Examples of such variants are also available in protein libraries under accession number given in Table 2.

**Table 2: Acession numbers of PP1 isoforms from other species**

| | PPP1CA | PPP1CB | PPP1CC |
|---|---|---|---|
| Mouse | NM_031868.1 | NM_172707.1 | NM_013636.2 |
| Rat | NM_031527.1 | NM_013065.1 | XM_346435.1 |
| Dog | NM_001003064.1 | NM_001003034.1 | NM_001003033.1 |

PP1 from other mammalian species have also been cloned and examples of Reference sequence accession numbers are given in Table 2. It should be understood that additional naturally-occurring PP1 variants or isoforms may be identified using conventional techniques,

### PP1 Inhibitors

The term PP1 inhibitor designates any compound or treatment that inhibits (e.g., reduces, abolishes or suppresses), either temporarily or permanently, the activity of a PP1 polypeptide. Such inhibitors are preferably but not always selective, i.e., they preferentially inhibit PP1 and essentially do not inhibit directly or do not bind other Protein Phosphatases. PP1 inhibitors may inhibit the biological activity of PP1 (e.g., its effect on protein de-phosphorylation), its binding to tra2-beta1 or to any tra2-betal-containing complex. They may also inhibit PP1 expression (e.g., transcription or translation), maturation, etc.

In a preferred embodiment, a PP1 inhibitor is a small drug compound. Such compounds have been reported in the literature, and include, by way of illustration, Tautomycin, Tautomycetin, Fostriecin, Microcystin-LR, Calyculin A, Cantharidin, Thyrsiferyl-23-acetate, Nodularin, Dephostatin, 3,4-Dephostatin, Fumonisin B1 and Motuporin, salts, optically active isomers and derivatives thereof, as well as any combination thereof. A particularly preferred PP1 inhibitor is Tautomycin, as well as salts and derivatives thereof.

It should be understood that other types of inhibitors may be used, such as decoys, peptides, inhibitory nucleic acids, intrabodies, single chain antibodies (ScFvs), etc.

More generally, PP1 inhibitors suitable for use in the present invention may be selected or validated using a number of tests, such as screening assays disclosed in the present application.

The inhibitors may be formulated in any form suitable for pharmaceutical use. This includes isotonic solutions, buffered solution, powder, lyophilized forms, gels, pastc, etc. The excipient and/or carriers may be selected from salts, stabilizing agents, etc. Examples of such excipients include, without limitation, water; alcohols, such as ethanol and methanol ; Chloroform ; DMSO ; phosphate buffers, sodium chloride, polymers, high molecular weigh proteins, etc., as well as combination(s) thereof. Particular excipients for use with the above disclosed PP1 inhibitors are listed in Table 3 below, together with suitable dosages. Furthermore, the PP1 inhibitors may be administered according to various schedules and routes, which may be adapted by a person skilled in the art. In particular, suitable administration routes include intravenous, intraarterial, intramuscular, intraperitoneal, intrathecal, transdermic or intra-organ injection. Oral or topical administrations may also be performed, depending on the inhibitor and disease.

**Table 3. Examples of in vivo administration conditions of PP1 inhibitors.**

| **Compound** | **In vivo concentration (range)** | **Excipient(s)** |
|---|---|---|
| Tautomycin | 5-600nM | Ethanol, Chloroform, Methanol |
| Tautomycetin | 1-10nM | DMSO |
| Fostriecin | 3-150µM | Water, Ethanol, Methanol |
| Microcystin-LR | 2-20nM | DMSO, Ethanol, Methanol |
| Calyculin A | 0,1-10nM | DMSO, Ethanol |
| Cantharidin | 1-10µM | DMSO, Ethanol |
| Thyrsiferyl-23-acetate | 0,1-10µM | DMSO |
| Nodularin | 2-20nM | Methanol:Water |
| Dephostatin | 1-7µM | DMSO, Chloroform |
| 3,4-Dephostatin | 2-18/µM | DMSO |
| Fumonisin B1 | 1-100µM | DMSO |
| Motuporin | 1-100µM | DMSO |

### Pharmaceutical uses

As mentioned before, the present invention relates to the use of a PP1 inhibitor for the manufacture of a medicament (drug) to treat a disease caused by improper pre-mRNA processing, as well as to corresponding methods of treatment.

Within the context of this invention, the term treatment includes preventive or curative therapy, and encompasses a complete, partial or local amelioration of a subject condition, including a reduction in the disease symptoms, a reduction in disease progression, a regression, etc. The term "treatment" particularly includes a reduction of the effects caused by improper pre-mRNA splicing.

The present invention is particularly suited to treat a disease caused by an improper pre-mRNA splicing which is regulated by a tra2-beta1-containing splicing enhancer complex. A number of diseases are caused by aberrant use of exons that are regulated by the splicing factor tra2-betal (Stoilov et al., 2004). Such diseases include, more preferably, neuro-degenerative diseases, mental disorders, tauopathies, cancers and cystic fibrosis.

A preferred example of a neuro-degenerative disease that can benefit from the present invention is spinal muscular atrophy. Spinal muscular atrophy is a neurodegenerative disorder with progressive paralysis caused by the loss of alpha-motor neurons in the spinal cord. The incidence is 1:6,000 live births and the carrier frequency 1 in 40, making SMA the second most common autosomal recessive disorder and the most frequent genetic cause of infantile death. SMA is caused by the loss of the SMN1 gene that encodes the SMN protein, which regulates snRNP assembly. Humans posses an almost identical gene- SMN2, that was generated through a recent dublication. Although both genes are almost identical in sequence, due to a translationally silent C->T change at position 6 in exon 7, they have different splicing pattern and exon 7 is predominantly excluded in SMN2. This exon skipping event generates a truncated, less stable and probably non-functional protein. Therefore, SMN2 cannot compensate the loss of SMN 1. The SMN protein functions in the assembly of snRNPs, Although in SMA patients the SMN protein is almost completely absent from all cells, for unknown reasons, alpha motoneurons are most severely affects and die, which causes the muscular atrophy. The disease can manifest in four phenotypes (type I to IV) that differ in onset and severity. The phenotypes correlate roughly with the number of SMN2 copies in the genome, most like because more SMN2 copies produce more SMN protein. Since stimulation of SMN2 exon7 usage would increase SMN protein levels and potentially cure the disease, work has concentrated on understanding the regulation of exon 7. As for CFTR exon 9 and 12, multiple factors determine the regulation, including a suboptimal polypyrimidine tract (Singh et al., 2004c), a central tra2-betal-dependent enhancer (Hofmann et al., 2000) and the sequence around the C->T change at position 6 that can either bind to SF2/ASF or hnRNPA1 (Cartegni and Krainer, 2002; Kashima and Manley, 2003). Recent large scale mutagenesis studies indicate that again a composite regulatory exonic element termed exict (extended inhibitory comtext) is responsible for the regulation of exon 7 inclusion (Singh et al., 2004a; Singh et al., 2004b). As illustrated in the examples, by regulating the improper alternative splicing, the invention can be used to treat SMA.

Tauopathies designate a group of diseases caused by the formation of neurofibrillary tangles (NFTs) (Mann and Pickering-Brown, 2001). Tau is a protein enriched in axons of mature and growing neurons. The tau transcript contains at least 16 exons, of which 8 are alternatively spliced, which leads to the expression of multiple isoforms in the brain. Tau protein associates with microtubules (MT), cytoskeletal structures whose primary function is to generate specific cell morphologies and organize intracellular components. In the adult human brain, the function of tau protein is regulated by insertion of protein sequences due to alternative splicing and by phosphorylation. Since the alternatively spliced exon 10 encodes a microtubule binding site, alternative splicing regulates the formation of tau proteins containing either 4 or 3 microtubule binding domains (4R, 3R). Adult human neurons have a characteristic ratio of these isoforms and pertubation of the correct ratio leads to the formation of neurofibrillary tangles (NFTs) and cell death. Pathological hyperphosphorylation of tau results in the dissociation of tau from microtubules and also leads to the formation of neurofibrillary tangles (NFTs). Hyperphosphorylated tau is a major component of NFTs. Once NFTs are formed, tau no longer binds to and stabilizes the neuronal cytoskeleton, which contributes to neuronal malfunction and death. Therefore, NFTs are a hallmark of several neurodegenerative diseases that are commonly names "tauopathies" (Mann and Pickering-Brown, 2001). They include progressive supranuclear palsy (PSP), corticobasal degeneration (CBD), Pick's disease (PiD) and frontotemporal dementia and parkinsonism linked to chromosome 17 (FTDP-17). Importantly, NFTs are one of the major neuropathological features of Alzheimer's disease (AD). NFTs are thought to contribute to neuronal cell dysfunction and death. The NFT burden is closely related to the decrease in neuronal population and to the intensity of dementia. NFTs are systematically observed in the normal population aged over 75 years.
The present invention now offers a novel approach to the effective treatment of tauopathies, particularly Alzheimer disease, progressive supranuclear palsy (PSP), corticobasal degeneration (CBD), Pick's disease (PiD), frontotemporal dementia, Parkinsonism linked to chromosome 17 (FTDP-17), neuronal cell dysfunction and death, dementia and ageing

Cystic Fibrosis (CFTR) is caused by 84 nt exon inclusion, which is regulated by the splicing factor tra2-beta1 (Stoilov et al., 2004). As demonstrated in the examples, this aberrant splicing can be corrected using the present invention, thereby treating such disease.

Cancers are also often linked to the presence of aberrant splicings. The invention is thus also appropriate to treat such cancers, including solid or liquid cancers, in particular those in which an improper splicing regulated by the splicing factor tra2-betal is observed.

### Drug Screening

The invention stems from the unexpected and important discovery that PP1 regulates improper splicing events in a number of pathological conditions. Accordingly, PP1 represents a valuable target for drug screening and development.

A further aspect of the invention also relates to methods of selecting or identifying or improving drugs that regulate pre-mRNA processing, the method comprising a step of determining whether a drug candidate inhibits PP 1.

A further embodiment of this invention relates to methods of selecting or identifying or improving a drug candidate for treating cancer or a CNS disorder or a tauopathy or Cystic Fibrosis, the methods comprising a step of determining whether said drug candidate inhibits PP 1.

Preferably, the methods are performed in vitro or ex vivo.

PP1 inhibition may be assessed in a number of ways, including using binding or cell-based assays. The method typically comprises contacting the drug candidate with PP 1 or a portion thereof, and determining whether the drug candidate binds to PP 1 or said portion thereof, such binding being an indication that the drug is a PP1 inhibitor. If required, the inhibitory activity or the selectivity may be further confirmed in additional assays, such as in vivo assays or in vitro tests as disclosed in the examples. Most preferably, the method is performed in the presence of tra2-betal or a PP1-binding domain thereof, and comprises determining whether the drug candidate interferes with the binding of PP 1 to tra2-beta1 or said binding domain thereof. PP 1 binds to an RVXF motif that is found in the splicing regulatory proteins tra2-betal, SF2/ASF, SRp30c and SRP54. This motif is evolutionary conserved among these proteins (Figure 6).
Multiple domains of PPP1C are involved in binding to substrates. Crystal structure analysis showed that this RVXF motif bound in an extended conformation to a hydrophobic channel within the C-terminal region of PPP1CC that is formed at the interface of the two beta-sheets of the beta-sandwich opposite to the catalytic-site channel. Three residues of the motif are incorporated into one of the PPP1CC beta-sheets as a sixth beta-strand parallel to the edge beta-strand (b- 14) (Barford, 1999).

In a specific embodiment, the PP1-binding domain of tra2-beta1 is a polypeptide fragment of said protein comprising at least the motif RVXF or a derivative thereof. In an other particular embodiment, the portion of PP1 as used in the screening assay comprises the C-terminal region thereof.

Further advantages and aspects of this invention will be disclosed in the following examples, which should be regarded as illustrative and not limiting the scope of this invention.

### Examples

### Example 1. Tra2-beta1 binds to PP1

3 x 10⁶ HEX293 cells were transfected with EGFP-tra2-beta1 and an EGFP-tra2-beta1 mutant (EGFP-tra2-beta1 RATA) that does not bind PP1. The transfection was performed using standard CaPhosphate method. The method is described in Protocol 1. Immunoprecipitation shows that only tra2-beta1 binds to PP1, i.e. that the interaction between the molecules is dependent on the PP I binding motif present in tra2-beta1. The tra2-betal RATA mutant is no longer able to bind to PP1. Immunoprecipitation was done with anti-GFP (Sigma) and WB with rabbit anti-PP1 antibody (1:5000). Denatured protein samples were resolved by 12% SDS-PAGE and transferred to nitrocellulose membrane by wet blotting. Immunostaining of the membranes and detection of the signals was performed with ECL solutions. Chemiluminescence reagent was carried out according to standard protocols. Immunoprecipitations are described in Protocol 2.
The results are presented in Figures 1 and 7. They show that only tra2-betal binds to PP1, i.e. that the interaction between the molecules is dependent on the PP1 binding motif present in tra2-beta1. The tra2-betal RATA mutant is no longer able to bind to PP1. EGFP-tagged rSLM2 protein was used in this assay as a negative control.
This example thus demonstrates the principle that PP1 binds in a complex with tra2-beta1 and regulates its protein interactions.

Protocol 1: Transient transfection using calcium phosphate
* Transient transfection of adherent HEK293 cells is performed using the calcium phosphate method on 35 mm plates (six-well tissue culture plate). The day before transfection, 3.0 x10⁵ cells per plate are seeded in 3 ml DMEM/10% FCS. This leads to approximately 40-60% confluency on the day of transfection. After splitting, the cells are incubated at 37°C in 5% CO₂ for 17-24h.
* Splicing assays are based on the titration of increasing amounts of plasmid DNA expressing a splicing factor to a constant concentration of minigene DNA. To avoid "squelching" effects, the 'empty' parental expression plasmid containing the promotor is added to ensure a constant amount of transfected DNA (Figure 2 A).
* The standard assay employs five reactions, each containing 1-2 µg of minigene DNA and an increasing amount of plasmid DNA expressing a splicing factor. The amount of minigene used has to be optimized in several trial experiments. 0, 0.5, 1, 1.5 and 2 µg of splicing factor DNA is a good starting point for the titration of splicing factors. The appropriate amount of empty vector (2, 0.5, 1, 1.5 and 0 µg) is added to ensure that equal amounts of DNA are transfected. The DNA solutions are brought to a total volume of 75 µl with water and 25 µl I M CaCl₂ are added. While mixing the DNA/CaCl₂ solution with a vortex, 100 µl of 2 x HBS is added dropwise. 1M CaCl₂ solution: Dissolve 5.4g CaCl₂·6H₂O in 20ml H₂O, sterilize by filtration, store at -20°C; 2 xHBS: Dissolve 1.6g NaCl (280mM), 0.074g KCl (10mM), 0.027g Na₂HPO₄·2H₂O (1.5mM), 0.2g dextrose (12mM) and 1g HEPES (50mM) in 90ml H₂O. Adjust pH to 7.05 with NaOH, then bring up to a total volume of 100ml with H₂O. Sterilize by filtration, store at -20°C.
* The mixture is incubated for 10-20 min at room temperature to allow the calcium phosphate-DNA precipitate to form.
* The precipitates are resuspended by pipetting and the complete mixture is added dropwise to the cultured cells.
* The dishes are incubated at 37°C in 3% CO₂ overnight.
* After the incubation, a fine precipitate is visible on the cells. The transfection efficiency can be estimated by fluorescence microscopy if an EGFP-tagged construct is used and should be at least 50% with HEK293 cells. If the splicing factor itself is not EGFP tagged, the use of pEGFP-C2 (Clontech, Heidelberg, Germany) as an 'empty' vector can help to monitor the transfection.

### Protocol 2: Immunoprecipitations

The day before transfection, 3.0 x10⁵ HEK293 cells per 3.5cm plate were seeded in 3 ml DMEM/10% FCS and incubated at 37°C in 5% CO₂ for 17-24h. Transient transfections of adherent HEK293 cells with 1µg of total cDNA (EGFP-tra2-beta1, HA-PP1) constructs were performed using the calcium phosphate method, see protocol 1. Cells were lysed in 200 µl RIPA buffer (1 % NP40, 1 % sodium deoxycholate, 0.1 % SDS, 150 mM NaCl, 10mM Na-phosphate pH 7.2, 2 mM EDTA, 50 mM NaF, 5 mM β-glycerolphosphate and freshly added 4 mM sodium orthovanadate, 1 mM DTT, 1 mM PMSF, 20 µg/ml aprotinine, and 100 U/ml benzonase) for 30 minutes on ice. Precipitates were cleared by centrifugation. 50 µl of this total cellular lysate were transferred into a 1.5ml tube, mixed with an equal amount of lxLaemmli buffer, boiled, and stored at -20°C overnight. 150 µl of the lysate were diluted fourfold in RIPA rescue (10 mM Na-phosphate pH 7.2, 1 mM NaF, 5 mM □-glycerolphosphate, 20 mM NaCl and freshly added 2 mM sodium orthovanadate, 1 mM DTT, 1 mM PMSF and 20 µg/ml aprotinine). Immunoprecipitations were performed overnight at 4°C with agitation using anti-GFP antibody (Boehringer) and protein A scpharose (Pharmacia) (protein A sepharose: scpharose 1:1, resuspended in RIPA rescue buffer), followed by three washes in HNTG (50mM HEPES pH7.5, 150mM NaCl, 1mM EDTA, 10% glycerol, 0.1% Triton X-100 and freshly added 2mM sodium orthovanadate, 100 mM NaF, 1 mM PMSF and 20 µg/ml aprotinine) (Nayler et al., 1997). The proteins were subsequently analyzed on SDS-PAGE followed by Western blotting and ECL (NEN) using anti-tra2 (Daoud et al., 1999), and anti-GFP (monoclonal: Boehringer-Mannheim, polyclonal: Clontech).

### Example 2. Tra2-beta1 dependent splicing enhancers are regulated by PP1

Three tra2-beta1 dependent exonic enhancers were tested: exon 2 of tra2-beta1, exon 7 of SMN2 and exon 10 of tau. Either PP1 or its nuclear inhibitor NIPP1 was cotransfected with these reporter genes. The splicing patterns were analysed by RT-PCR and quantitated.
Three tra2-beta1 dependent exonic enhancers were tested: exon 2 of Tra2-beta1, exon 7 of SMN2 and exon 10 of Tau. Either PP1 or its nuclear inhibitor NIPP1 was cotransfected in increased concentration (1, 2 and 4µg) with these reporter genes.
*In vivo* splicing assays were performed as described in Protocol 3. In brief, 1 µg of the indicated wild-type *pSMN* minigene, MGTra minigene that spans the first four exons of the gene Tra2beta and human SV9/10L/11 exon 10 tau MG were used together with increasing amounts of pEGFP-PP1gammal and pEGFP-NIPP1. The appropriate amount of empty pEGFP-C2 vector was added to ensure that equal amounts of DNA were transfected. HEK293 cells were transfected by using calcium phosphate method. The splicing patterns were analysed by RT-PCR and quantitated. RNA was isolated 24h (HEK293) posttransfection by using the RNeasy total RNA kit (Qiagen, Hilden, Germany) and transcribed into cDNA Reverse transcription was performed in a total volume of 8.5µl by using 2µl RNA, 1µl oligo (dT) (0.5mg/ml), lx first-strand buffer, 100mM DTT, 10mM dNTP, 50units SuperScript II RT, and 7units RNase inhibitor at 42°C for 45min. To ensure that only plasmid-derived *SMN,* Tra2 beta or Tau transcripts were detected, subsequent amplification was performed with a vector-specific forward primer (pCI-forw: 5'-GGT GTC CAC TCC CAG TTC AA, SEQ ID NO: 1) and the *SMN*-specific reverse primer SMNex8-rev (SMNex8-rev: 5'-GCC TCA CCA CCG TGC TGG, SEQ ID NO: 2) for SMN, for reverse transcription of Tra2beta: a vector specific primer pCR-RT-reverse (GCCCTCTAGACTCGAGCTCGA, SEQ ID NO: 3) for the following PCR amplification primers, MGTra-F-Bam and MGTra-R-Xho were used that annealed to both the endogenous and transfected human Tra2beta cDNA, and for the tau SV9/10L/11 minigene, reverse transcription followed by PCR using the primers sense INS 1 (cag cta cag teg gaa acc atc age aag cag) and antisense INS3 (cac ctc cag tgc caa ggt ctg aag gtc acc) was performed.
PCR products were then separated on agarose and PA gels and quantified using Image Quant (AP Biotech). All the experiments were repeated at least 3 times.
The results are presented on Figure 2. They demonstrate the principle that tra2-betal-dependent splicing enhancers are regulated by PP1.

Protocol 3: Analysis of splicing patterns derived from Minigenes
- RNA is isolated 17-24 hours after transfection using an RNeasy mini kit (Qiagen, Hilden, Germany) following the manufacturer's instructions. RNA is eluted in 40□1 RNAse free H₂O. The optimal transfection time has to be determined empirically.
   It is important to quantify the mRNA. 4 µl of the RNA are dissolved in 1 ml of water, the OD at 260 nm is measured. The value obtained is multiplied by 10 and this reading gives the concentration in µg/µl.
- Best results are achieved when reverse transcription and PCR are performed immediately after the RNA purification, thus avoiding freezing of the RNA or reverse transcription reaction.
- For reverse transcription, 1 µg of isolated RNA are mixed with 5 pmol antisense minigene specific primer in 0.5 µl H₂O, 2 µl 5xRT buffer, 1 µl 100 mM DTT, 1 µl 10 mM dNTP, 3 µl H₂O, 0.25 µl RNase inhibitor and 0.25 µl H- reverse transcriptase (Superscript, Promega). In one sample the RNA is substituted with water as a control. After a brief centrifugation, the tubes are incubated for 45 min in a 42°C water bath. Optionally 0.2µl *DpnI* (New England Biolabs) can be included in each sample to destroy vector DNA.
- During this incubation period, the PCR mixture is prepared. It consists of 50 pmol of sense and antisense primer each, 100 µl 10x PCR buffer, 20 µl 10 mM dNTPs in a total of 1000 µl water. The optimal MgCl₂ concentration for amplification has to be determined empirically in trial experiments and is usually in a range of a final concentration of 1.5 -3.0 mM.
- For six reactions, 1 µl Taq polymerase is added to 300 µl PCR mixture. 2 µl of the RT reaction are added to 50 µl of this mix and PCR is performed.
- The PCR program must be optimized for each minigene in trial experiments as we found that often identical programs show variations of amplification products when different thermocycler models are used. Using the Gene Amp PCR Systems 9799 from Perkin Elmer Applied Biosystems, we use the following profile for the SMN2 minigene (Figure 2): Initial denaturation for 4 minutes at 94°C; 30 cycles: 20 seconds denaturation at 94°C, annealing at 62°C for 20 seconds, extension at 72 °C for 20 seconds, after 30 cycles final extension at 72°C for 5 min and cooling to 4°C. This program was used as a starting point when optimizing a new reaction.
- The PCR reaction products are analyzed on a 0.3-0.4 cm thick 2% agarose TBE gel.

### Example 3. The PP1 inhibitor tautomycin regulates tra2-betal dependent enhancer

HEK293 cells were transfected with wild type SMN2 MG or tra2-beta1 dependent reporter genes and treated with 5-300nM oftautomycin or the substances listed in Table 1. Cells were treated 13 hours after transfection. RNA was isolated 18 h after transfection using an RNAesy mini kit (Qiagen), following the manufacturer's instructions.

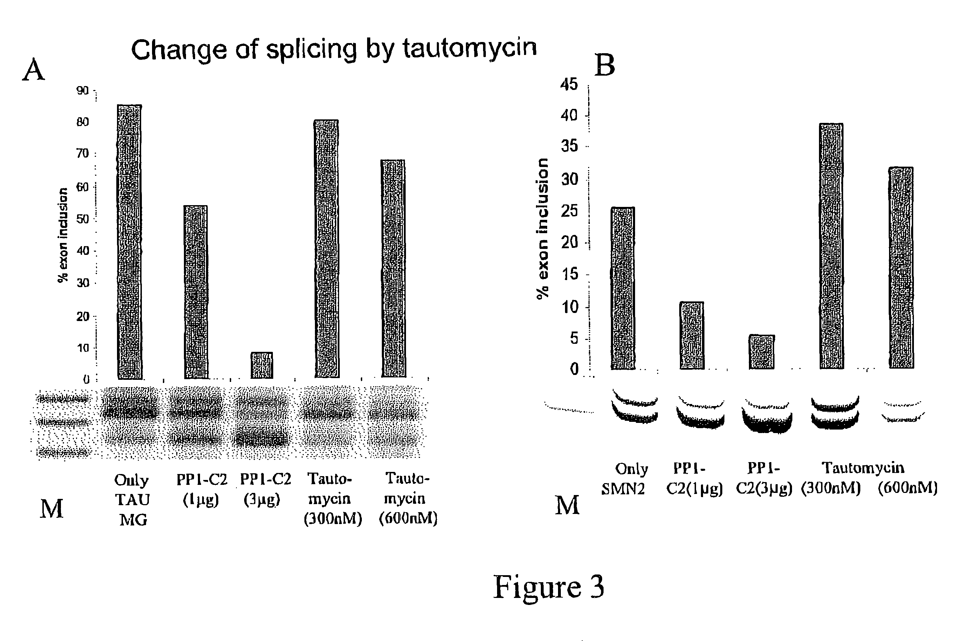

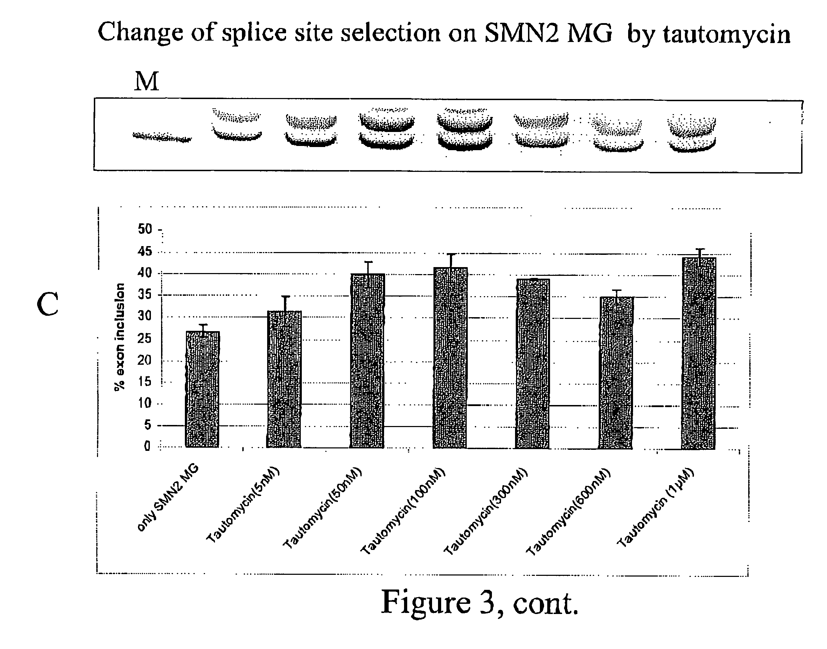

The results of these experiments are depicted Figure 3B and show that tra2-betal-dependent splicing enhancers can be manipulated by the drug tautomycin.

### Example 4. The PP1 inhibitors also regulate the tra2-beta1 dependent enhancer

The PP1 inhibitors, analogues of tautomycin: Cantharidin (Sigma), Calyculin A (Biomol), Microcystin ―LR (Axxora) and Dephostatin (Calbiochem) promote the exon 7 inclusion of SMN2 MG by inhibition of PP1. Cells (3X10 ⁵) were transferred in 6xwell dishes and transfected with 1 µg of pSMN2 wild type MG. The treatment of HEK 293 cells was for 13 hours for each inhibitor after transfection. The concentration for Cantharidin is - 0,1mkM to3mkM, for Dephostatin: 1-3mkM, for Microcystin: 2nM to 10nM, for Calyculin A: 0,1nM to 2nM.
The results of this experiment are presented Figure 3A and show that tra2-beta1-dependent splicing enhancer can also be manipulated by different PP 1 inhibitors.

### Example 5. The PP1 inhibitor tautomycin promotes SMN protein expression in SMA derived patient human fibroblasts.

Human patient un-transformed SMA1 fibroblasts GM03815 from NIGMS family 553 (heterozygous with SMA type I) and a healthy GMO357 with Metachromatic Leucodystrophy, Late-Infantile (provided by Coriell Cell Repository, NJ, USA) were transferred (2x10⁵) into 6 well dishes using DMEM medium supplemented with 10% FCS. Tautomycin (Calbiochem) was dissolved in ethanol (99%) and added dropwise for a final concentration of 5-40nM 24 hours posttransfection. For each experiment, to one of the dishes only ethanol was added to serve as a control. VPA in concentration 3 µM was used as a positive control. Cells were incubated for 1 to 5 days at 5% CO₂ and 37°C. Prior to lysis in 150µl RIPA buffer, cells were washed in lx PBS buffer. The level of SMN protein expression was identified by WB with a specific mouse monoclonal anti-SMN antibody 7B10 (1:700). The equal loading of the protein was proved by mouse anti-GAPDH antibody (1:2000). Denatured protein samples were resolved by 12% SDS-PAGE and transferred to nitrocellulose membrane by wet blotting. Immunostaining of the membranes and detection of the signals was performed with ECL solutions.
Chemiluminescence reagent was carried out according to standard protocols.

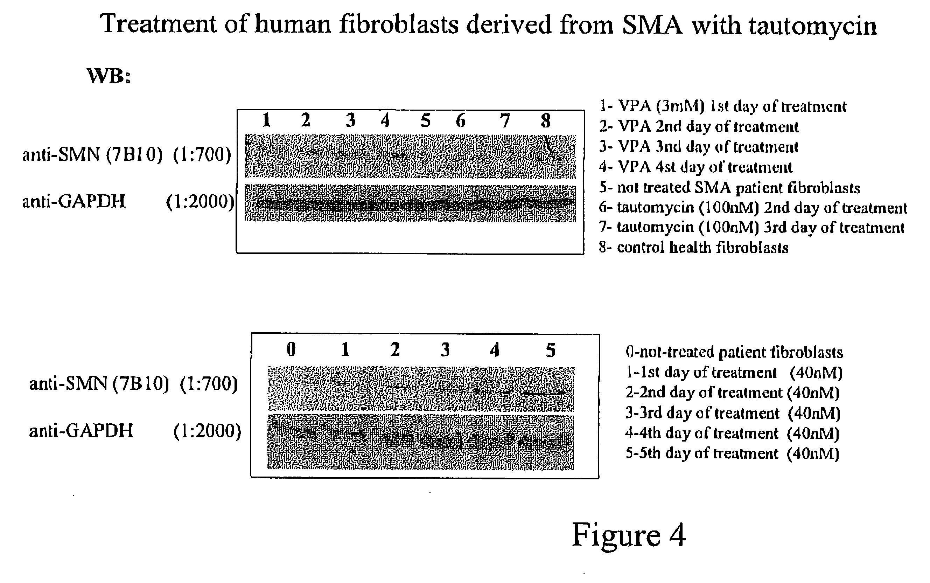

The results of this experiment are presented in Figures 4 and 5, and show that low molecular weight PP inhibitors can correct the splicing pattern of the endogenous human SMN2 gene *in vivo.*

### References

Anderson, S-L., Qiu, J. and Rubin, B.Y., 2003. EGCG corrects aberrant splicing of IKAP mRNA in cells from patients with familial dysautonomia. Biochem Biophys Res Commun 310, 627-33.
Andreassi, C., Jarecki, J., Zhou, J., Coovert, D.D., Monani, U.R., Chen, X., Whitney, M., Pollok, B., Zhang, M., Androphy, E. and Burghes, A.H., 2001. Aclarubicin treatment restores SMN levels to cells derived from type I spinal muscular atrophy patients. Hum Mol Genet 10, 2841-9.
Asparuhova, M., Kole, R. and Schumperli, D., 2005. Antisense derivatives of U7 and other small nuclear RNAs as tools to modify pre-mRNA splicing patterns. Gene Ther. Regualtion in press.
Barford, D., 1999. Colworth Medal Lecture. Structural studies of reversible protein phosphorylation and protein phosphatases. Biochem Soc Trans 27, 751-66.
Barker, H.M., Brewis, N.D., Street, A.J., Spurr, N.K. and Cohen, P.T., 1994. Three genes for protein phosphatase I map to different human chromosomes: sequence, expression and gene localisation of protein serine/threonine phosphatase 1 beta (PPP1CB). Biochim Biophys Acta 1220, 212-8.
Black, D.L., 2003. Mechanisms of Alternative Pre-Messenger RNA Splicing. Annu Rev Biochem 291-336.
Brichta, L., Hofmann, Y., Hahnen, E., Siebzehnrubl, F.A., Raschke, H., Blumcke, I., Eyupoglu, I.Y. and Wirth, B., 2003. Valproic acid increases the SMN2 protein level: a well-known drug as a potential therapy for spinal muscular atrophy. Hum Mol Genet 12, 2481-9.
Carstens, R.P., McKeehan, W.L. and Garcia-Blanco, M.A., 1998. An intronic sequence element mediates both activation and repression of rat fibroblast growth factor receptor 2 pre-mRNA splicing. Mol Cell Biol 18, 2205-17.
Cartepni, L. and Krainer, A.R., 2002. Disruption of an SF2/ASF-depeadent exonic splicing enhancer in SMN2 causes spinal muscular atrophy in the absence of SMN1. Nat Genet 30, 377-84.
Cartegni, L. and Kraincr, A.R., 2003. Correction of disease-associated exon skipping by synthetic exon-specific activators. Nat Struct Biol 10,120-5.
Celotto, A.M. and Graveley, B.R., 2001. Alternative splicing of the Drosophila Dscam pre-mRNA is both temporally and spatially regulated. Genetics 159, 599-608.
Celotto, A.M. and Graveley, B.R., 2002. Exon-specific RNAi: a tool for dissecting the functional relevance of alternative splicing. Rna 8, 718-24.
Chang, J.G., Hsieh-Li, H.M., Jong, Y.J., Wang, N.M., Tsai, C.H. and Li, H., 2001. Treatment of spinal muscular atrophy by sodium butyratc. Proc Natl Acad Sci U S A 98,9808-13.
Chao, H., Mansfield, S.G., Bartel, R.C., Hiayanna, S., Mitchell, L.G., Garcia-Blanco, M.A. and Walsh, C.E., 2003. Phenotype correction of hemophilia A mice by spliceosomc-mediated RNA trans-splicing. Nat Med 9, 1015-9.
Garcia-Blanco, M.A., Baraniak, A.P. and Lasda, E.L., 2004. Alternative splicing in disease and therapy. Nat Biotechnol 22, 535-46.

Hofmann, Y., Lorson, C.L., Stamm, S., Androphy, E.J. and Wirth, B., 2000. Htra2-beta 1 stimulates an exonic splicing enhancer and can restore full-length SMN expression to survival motor neuron 2 (SMN2). Proc Natl Acad Sci U S A 97, 9618-23.
Johnson, J.M., Castle, J., Garrett-Engele, P., Kan, Z., Loerch, P.M., Armour, C.D., Santos, R., Schadt, E.E., Stoughton, R. and Shoemaker, D.D., 2003. Genome-wide survey of human alternative pre-mRNA splicing with exon junction microarrays. Science 302, 2141-4.

Kampa, D., Cheng, J., Kapranov, P., Yamanaka, M., Brubaker, S., Cawley, S., Drenkow, J., Piccolboni, A., Bekiranov, S., Helt, G., Tammana, H. and Gingeras, T.R., 2004. Novel RNAs identified from an in-depth analysis of the transcriptome of human chromosomes 21 and 22. Genome Res 14, 331-42.
Kashima, T. and Manley, J.L., 2003. A negative element in SMN2 exon 7 inhibits splicing in spinal muscular atrophy. Nat Genet 34, 460-3.
Lacerra, G., Sierakowska, H., Carestia, C., Fucharoen, S., Summerton, J., Weller, D. and Kole, R., 2000. Restoration of hemoglobin A synthesis in erythroid cells from peripheral blood ofthalassemic patients. Proc Natl Acad Sci USA 97, 9591-6.
Lan, N., Howrey, R.P., Lee, S.W., Smith, C.A. and Sullenger, B.A., 1998. Ribozyme-mediated repair of sickle beta-globin mRNAs in erythrocyte precursors. Science 280, 1593-6.
Liu, S., Asparuhova, M., Brondani, V., Ziekau, I., Klimkait, T. and Schumperli, D., 2004. Inhibition of HIV-1 multiplication by antisense U7 snRNAs and siRNAs targeting cyclophilin A. Nucleic Acids Res 32, 3752-9.
Lopez-Bigas, N., Audita, B., Ouzounis, C., Parra, G. and Guigo, R., 2005. Are splicing mutations the most frequent cause of hereditary disease? FEBS Lett in press.
I,unn, M.R., Root, D.E., Martino, A.M., Flaherty, S.P., Kelley, B.P., Coovert, D.D., Burghes, A.H., Man, N.T., Morris, G.E., Zhou, J., Androphy, E.J., Sumner, C.J. and Stockwell, B.R., 2004. Indoprofen upregulates the survival motor neuron protein through a cyclooxygenase-independent mechanism. Chem Biol 11, 1489-93.
Maniatis, T. and Reed, R., 2002. An extensive network of coupling among gene expression machines. Nature 416, 499-506.
Maniatis, T. and Tasic, B., 2002. Alternative prc-mRNA splicing and proteome expansion in metazoans. Nature 418, 236-43.
Mann, C.J., Honeyman, K., Cheng, A.J., Ly, T., Lloyd, F., Fletcher, S., Morgan, J.E., Partridge, T.A. and Wilton, S.D., 2001. Antisense-induced exon skipping and synthesis of dystrophin in the mdx mouse. Proc Natl Acad Sci U S A 98, 42-7.
Mann, D.A. and Pickering-Brown, S., 2001. The status of "Pick's Disease" and other tauopathies within the frontotemporal dementias. Neurobiol Aging 22, 109-111.
Missler, M. and Sudhof, T.C., 1998. Neurexins: three genes and 1001 products. Trends Genet 14,20-6.
Pagani, F., Stuani, C., Tzetis, M., Kanavakis, E., Efthymiadou, A., Doudounakis, S., Casals, T. and Baralle, F.E., 2003. New type of disease causing mutations: the example of the composite exonic regulatory elements of splicing in CFTR exon 12. Hum Mol Genet 12, 1111-20.
Rogers, C.S., Vanoye, C.G., Sullenger, B.A. and George, A.L., Jr., 2002. Functional repair of a mutant chloride channel using a trans-splicing ribozyme. J Clin Invest 110, 1783-9.
Sazani, P. and Kole, R., 2003. Therapeutic potential of antisense oligonucleotides as modulators of alternative splicing. J Clin Invest 112, 481-6.
Singh, N.N., Androphy, E.J. and Singh, R.N., 2004a. An extended inhibitory context causes skipping of exon 7 of SMN2 in spinal muscular atrophy. Biochem Biophys Res Commun 315, 381-8.
Singh, N.N., Androphy, E.J. and Singh, R.N., 2004b. In vivo selection reveals combinatorial controls that define a critical exon in the spinal muscular atrophy genes. Rna 10, 1291-305.
Singh, N.N., Androphy, E.J. and Singh, R.N., 2004c. The regulation and regulatory activities of alternative splicing of the 5MN gene. Crit. Rev. Eukary. Gene Exp. 14, in press.
Skordis, L.A., Dunckley, M.G., Yue, B., Eperon, I.C. and Muntoni, F., 2003. Bifunctional antisense oligonucleotides provide a trans-acting splicing enhancer that stimulates SMN2 gene expression in patient fibroblasts. Proc Natl Acad Sci U S A 100, 4114-9.
Slaugenhaupt, S.A., Mull, J., Leyne, M., Cuajungco, M.P., Gill, S.P., Hims, M.M., Quintero, F., Axelrod, F.B. and Gusella, J.F., 2004. Rescue of a human mRNA splicing defect by the plant cytokinin kinetin. Hum Mol Genet 13, 429-36.
Smith, C.W. and Valcarcel, J., 2000. Alternative pre-mRNA splicing: the logic of combinatorial control. Trends Biochem. Sci. 25, 381-388.
Song, Q., Khanna, K.K., Lu, H. and Lavin, M.F., 1993. Cloning and characterization of a human protein phosphatase 1-encoding cDNA. Gene 129, 291-5
Soret, J., Bakkour, N., Maire, S., Durand, S., Zekri, L., Gabut, M., Fic, W., Divita, G., Rivalle, C., Dauzonne, D., Nguyen, C.-H., Jeanteur, P. and Tazi, J., 2005. Selective modification of alternative splicing by indole derivatives that target SR protein splicing factors. Proc Natl Acad Sci U S A in press.
Stamm, S., 2002. Signals and their transduction pathways regulating alternative splicing: a new dimension of the human genome. Hum. Mol. Genet. 11, 2409-2416.
Stoilov, P., Daoud, R., Nayler, O. and Stamm, S., 2004. Human tra2-beta1 autoregulates its protein concentration by influencing alternative splicing of its pre-mRNA. Hum Mol Genet 13, 509-524.
Watanabe, T. and Sullenger, B.A., 2000. Induction of wild-type p53 activity in human cancer cells by ribozymes that repair mutant p53 transcripts. Proc Natl Acad Sci U S A 97, 8490-4.
Xavier, K.A., Eder, P.S. and Giordano, T., 2000. RNA as a drug target: methods for biophysical characterization and screening. Trends Biotechnol 18, 349-56.
Zhang, M.L., Lorson, C.L., Androphy, E.J. and Zhou, J., 2001. An in vivo reporter system for measuring increased inclusion of exon 7 in SMN2 mRNA: potential therapy of SMA. Gene Ther 8, 1532-8.

## Claims

1. The use of a Protein Phosphatase I (PP1) inhibitor for the manufacture of a medicament to treat a disease caused by improper pre-mRNA processing.

2. The use of claim 1, wherein the improper pre-mRNA processing is regulated by a tra2-beta1-containing splicing enhancer complex.

3. The use of claim 1 or 2, wherein the disease is selected from neuro-degenerative diseases, tauopathies, cancers and cystic fibrosis.

4. The use of anyone of claims 1 to 3, wherein the disease is selected from spinal muscular atrophy, Alzheimer disease, progressive supranuclear palsy (PSP), corticobasal degeneration (CBD), Pick's disease (PiD), frontotemporal dementia, Parkinsonism linked to chromosome 17 (FTDP-17), neuronal cell dysfunction and death, dementia, ageing, cancers and cystic fibrosis.

5. The use of a PP1 inhibitor to influence splice site selection in a mammalian cell in vitro or ex vivo.

6. The use of any one of claims 1 to 5, wherein the inhibitor is a selective PP1 inhibitor.

7. The use of any one of claims 1 to 5, wherein the PP 1 inhibitor is a small drug, preferably selected from Tautomycin, Tautomycetin, Fostriecin, Microcystin-LR, Calyculin A, Cantharidin, Thyrsiferyl-23-acetate, Nodularin, Dephostatin, 3,4-Dephostatin, Fumonisin B1 and Motuporin, salls and derivatives thereof, as well as any combination thereof.

8. The use of any one of claims 1 to 6, wherein the PP1 inhibitor is used, simultaneously or sequentially, in combination with at least one additional active agent or treatment.

9. The use of tautomycin for the manufacture of a medicament to treat spinal muscular atrophy, tauopathies or Cystic fibrosis.

10. The use of a PP1 inhibitor for the manufacture of a medicament to treat the effects of cancer that are caused by the selection of wrong splice sites.

11. A method of selecting or identifying or improving a drug that regulates pre-mRNA processing, the method comprising a step of detennining in vitro or ex vivo whether a drug candidate inhibits PP1.

12. A method of selecting or identifying or improving a drug candidate for treating cancer or a CNS disorder or a tauopathy or Cystic Fibrosis, the method comprising a step of determining in vitro or ex vivo whether said drug candidate inhibits PP 1.

13. The method of claim 11 or 12, which comprises contacting the drug candidate with PP1 or a portion thereof, and determining whether the drug candidate binds to PP1 or said portion thereof, such drugs being selected.

14. The method of claim 13, wherein said contacting step is performed in the presence of tra2-beta1 or a PP1-binding domain thereof, and the method comprises determining whether the drug candidate interferes with the binding of PP1 to tra2-betal or said binding domain thereof.
